Europäisches Patentamt

European Patent Office

Office européen des brevets

(11) Publication number: **0 400 518 A2**

# EUROPEAN PATENT APPLICATION

(21) Application number: 90110051.1

(51) Int. Cl.5: **A61M 1/02**

(22) Date of filing: 28.05.90

(30) Priority: 02.06.89 SE 8902014

(43) Date of publication of application:
05.12.90 Bulletin 90/49

(84) Designated Contracting States:
BE CH DE DK ES FR GB IT LI NL

(71) Applicant: **GAMBRO AB**
**Post Box 10101**
**S-220 10 Lund(SE)**

(72) Inventor: **Bonnevier, Ulf**
**Västervangsgatan 11**
**S-217 74 Malmö(SE)**

(74) Representative: **Boberg, Nils Gunnar Erik**
**Gambro AB Patent Department Box 10101**
**S-220 10 Lund(SE)**

(54) Auto transfusion system for the collection, treatment and return of a patient's blood.

(57) An auto transfusion system for the collection, treatment and return of a patient's blood, comprising means for performing the respective steps, for example, a suction device (1) for drawing up blood and, where present, any rinsing fluids from an operation site or similar, a source (2) of anticoagulant, a source (2) of diluent, a collection vessel (3) for the to-be-treated blood, a filter (5) for the removal of a filtrate comprising a quantity of the diluent, and a storage vessel (8) for the treated blood.

The system according to the invention is primarily characterized in that the said sources consist of a common storage vessel (2) for a mixture of anticoagulants and diluents.

The system can further comprise a measuring device (9) for measuring the haematocrit- and/or Hb value of the treated blood.

*Fig. 1*

## AUTO TRANSFUSION SYSTEM FOR THE COLLECTION, TREATMENT AND RETURN OF A PATIENT'S BLOOD

TECHNICAL FIELD

The present invention relates to an auto transfusion system for the collection, treatment and return of a patient's blood, comprising means for performing the respective steps, for example a suction device for drawing up blood and, where present, any rinsing fluids from an operation site or similar, a source of anticoagulant, a source of diluent, a collection vessel for the to-be- treated blood, a filter for the removal of a filtrate comprising at least a quantity of a diluent, and a storage vessel for the treated blood.

The system is primarily intended to be used during operations, for example in connection with vascular surgery, liver operations and orthopedic operations, such as hip joint operations. It can, however, also be used post operatively, e.g. in intensive care wards after major operations.

BACKGROUND ARTS

Available systems for similar purposes, which are marketed, for example, under the name of Haemonetics Cell Saver, IBM Cell Washer and Dideco, all work with centrifuging and, after a careful wash, give a final product containing only red blood cells in a salt solution. These systems are also relatively expensive, owing to the centrifuges included. As an example of a system of this type, reference is made to the article "Autotransfusion and emergency surgery; preliminary report on an improved technique" in The International Journal of Artificial Organs, vol. 8, No. 4, 1985, pages 221-224.

Simpler systems also exist, which are sold, for example, under the name of Sorenson and Solcotrans. These systems consist quite simply of a plastic cannister, which is pre-filled with a certain amount of ACD-solution and is provided with a built-in filter. When the cannister has been filled with blood it is simply turned upside down, so that the blood can be re-transfused through the filter. These simpler systems, however, are not sufficiently effective in the case of greater haemorrages and, moreover, the return blood frequently also contains amount of washing liquids, anti-coagulants and other additives.

A more recent system is described for example in Swedish patent No. 453 360 and its corresponding European publication EP-A-223 126. The present invention concerns a further development of said system.

DISCLOSURE OF INVENTION

It is an object of the invention to provide a simple system of the type defined above, with the help of which it is possible to return wholly or partly cleaned whole blood, including its plasma and many other valuable substances, such as coagulation factors and the like.

The whole blood moreover will be concentrated into a suitable haematocrit value and/or Hb-value in that superfluous salt solution and anti-coagulant solution are filtered off.

The invention can be defined in various ways. Preferably it can be said to be characterized in that the above mentioned sources consist of a common storage vessel for a mixture of anticoagulant and diluent. If the anti-coagulant consists of ACD (Acetate Citrate Dextrose) Formula A, 2.2% (Travenol) and the diluent consists of a 0.9% NaCl solution, then an optimal mixture can be prepared right from the start by combining 4/5 diluent with 1/5 anticoagulant. In proportion to the blood, the mixing ratio between the various fluids should be: blood/ACD 2.2% = 1/5 and blood/NaCl 0.9% + ACD 2.2% = 1/1. Mixing the various constituents separately with the blood leads to difficulties with the dosage and the risk of an overdose of anti-coagulant (the citrate) is very likely. This in turn may lead to acute citrate poisoning. This risk is greatly reduced, or even totally eliminated, by using the system according to the present invention.

The said common storage vessel is preferably connected to the suction device or to a point near the suction device on a conduit leading therefrom. In this way a careful treatment of the blood is achieved, whilst at the same time the risk of coagulation is eliminated.

Preferably the said connection is achieved via a manually operable valve, which is so dimensioned that in its open position it gives a maximum mixing ratio between blood and added fluid of 1:1. A transgression of this value can give rise to coagulation and haemolysis. On the other hand a shortfall has less importance since superfluous fluid can be removed.

Suitably, in accordance with the above mentioned Swedish patent , a recirculation circuit is arranged between said collection vessel and the collection vessel for finally treated blood, such circuit comprising a filter, a recirculation pump and an intermediate storage vessel for partially treated blood. Within this recirculation circuit surplus fluid can be removed from the blood, whilst at the same time a careful treatment of the latter is obtained.

To ensure that the blood returned to the patient has an optimal composition, a measuring device is suitably arranged to measure the haematocrit and/or Hb-value of the treated blood. This measuring device is appropriately arranged in the recirculation circuit and preferably in direct contact with the recirculation pump.

A preferred embodiment of the system according to the invention is characterized by a valve arranged between said intermediate storage vessel and the storage vessel for finally treated blood, said valve being opened manually or automatically when the measured blood value indicates that the treated blood is suitable for returning to the patient.

According to another preferred embodiment of the auto transfusion system according to the invention, this comprises a suction device for drawing up blood and, where present, any rinsing fluids from an operation site or similar, a source of anticoagulant, a source of diluent, a collection vessel, a filter for the removal of a filtrate comprising at least a quantity of the diluent, and a storage vessel for finally treated blood. This embodiment is characterized by a measuring device for measuring the haematocrit- and/or Hb-value of the treated blood. This measuring preferably takes place continuously. In this way the treatment of the blood can be interrupted precisely when the blood is suitable for returning to the patient.

Further to this embodiment a recirculation circuit is suitably located between said collection vessel and the collection vessel for finally treated blood, said circuit comprising a filter, a recirculation pump and an intermediate storage vessel for partially treated blood. Said measuring device is appropriately arranged in the same recirculation circuit and preferably in direct contact with the recirculation pump.

Further to this embodiment a valve is suitably arranged between said intermediate storage vessel and the storage vessel for finally treated blood, said valve being opened manually or automatically when the measured blood value indicates that the treated blood is suitable for returning to the patient.

Similarly to the above discussed preferred embodiment the said mentioned sources can consist of a common storage vessel for a mixture of anticoagulant and diluent, whereby the said common storage vessel can be connected to the suction device or to a point near to the suction device on a conduit leading therefrom. This connection is suitably achieved by a manually operable valve which is so dimensioned that, in its open position, it gives a maximum mixing ratio between blood and added fluid of 1:1.

The above mentioned measuring device is appropriately based on a measuring of infrared light passing through the blood. The choice of this measuring method is a consequence of the measuring within this range being less dependent on the blood's oxygen content. As a sender, a galliumaluminum-arsenic-diode having a maximum intensity of approximately 880 nm can be used. Suitably the light of the diode is pulsated at a relatively high frequency, for example 33 kHz. Though this frequency is not critical, it should lie well above 50-100 Hz, a frequency range common to many interference sources. Since electro-acoustic phenomena could also create disturbance, the sender frequency should be selected above the normal audible range.

On the receiver side, the signal firstly is appropriately amplified in a current amplifier and then transmitted to a bandpass filter which is set to the same high frequency, for example 33 kHz. It is this combination of sender and filter, which reduces the possibility of interference.

Since the absorption in a sample occurs as an exponential function, a logarithmic amplifier is appropriately employed to obtain adequate signal sizes and a suitable measuring range.

The signal from the logarithmic amplifier is then suitably connected to another amplifier with offset-control. This control can be used to adjust the rest level of the amplifier's output. The output signal is suitably compared in a comparator with a reference value which can be adjusted as desired. When the signal from the amplifier exceeds the reference value, the comparator will switch and admit a signal if the blood is suitable for returning to the patient. The measured value and the reference value can be checked by means of a digital display.

The system according to the invention suitably consists of means for comparing the measured haematocrit value and/or Hb-value with a reference value which is preferably based on a blood sample taken from the patient before treatment and which is measured with the help of the same haematocrit- and/or Hb measuring device.

## BRIEF DESCRIPTION OF THE DRAWINGS

The invention will be described in more detail in the following with reference to the attached drawings which, by way of example, show schematically the system according to the invention and details of the same.

Fig 1 hereby shows a block diagram of the whole system.

Fig 2 shows a suction device in the system.

Fig 3-5 show an alternative embodiment of the valve construction of the suction device.

Fig 6 shows a block diagram of a device for measuring haematocrit- and/or Hb within the system.

Fig 7 shows the result of a measurement with the help of the device according to fig 6 and a comparison of this measurement with that of an existing marketed measuring device.

Fig 8 shows a simple sketch of the measuring device which is used for the measurements according to fig 7.

Fig 9 finally shows how the measuring device can be combined with the recirculation pump in the system.

PREFERRED MODES OF CARRYING OUT THE INVENTION

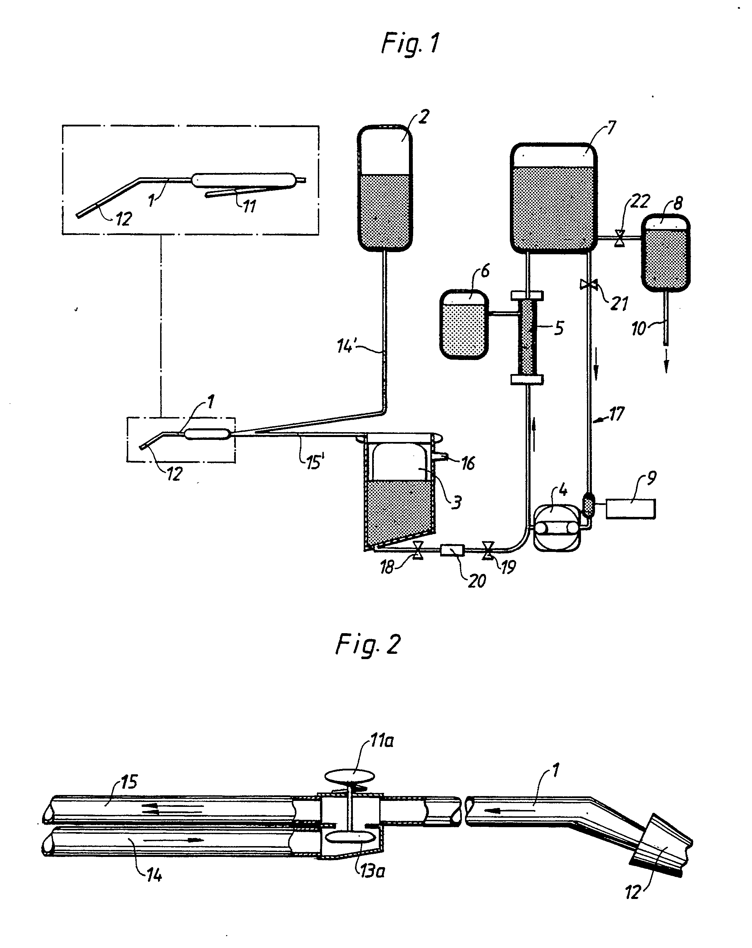

Fig 1 shows by way of example a preferred embodiment of the system according to the invention. Reference numeral 1 denotes a suction device for drawing up blood and any possible rinsing liquids from an operation site or similar. A mixture of anticoagulant and diluent is supplied to the suction device from a storage vessel 2 containing the said mixture. This mixture is, in turn, mixed in the suction device 1 with the drawn up blood and then led to a cardiotomy reservoir 3. By means of a pump 4 the whole mixture is drawn into a recirculation circuit 17 which further comprises a filter 5 and an intermediate storage vessel 7. With the help of the filter 5 surplus liquid is removed to a collection vessel 6. At the same time a substantial proportion of the anticoagulant, which can consist of for example heparin or ACD (Acetate Citrate Dextrose) is removed. The recirculation circuit further includes a device 9 for preferably continuously measuring the haematocrit or Hb value of the treated blood. When an appropriate level of one of these values is attained, the recirculation can be interrupted and the treated blood transferred to a final storage vessel 8 from where it is returned via a tube 10 to the patient. Preferably, this takes place intermittently, though it can be performed continuously.

An enlarged view of the suction device 1 is also depicted in fig 1. Reference numeral 11 denotes a regulating lever for an inlet valve in the suction device, by means of which the supply of the mixture from the storage vessel 2 can be controlled. Fig 2 shows an example of such controlling means. This figure schematically shows suction device 1 with its suction tip 12 and its regulating lever indicated by 11a. By means of the latter, the valve cone 13a can be moved between a completely closed position and at least one open position. This permits, when desired, the mixture of anticoagulant and diluent to be fed via conduit 14 into conduit 15 to allow its mixing with the blood drawn up by the suction device.

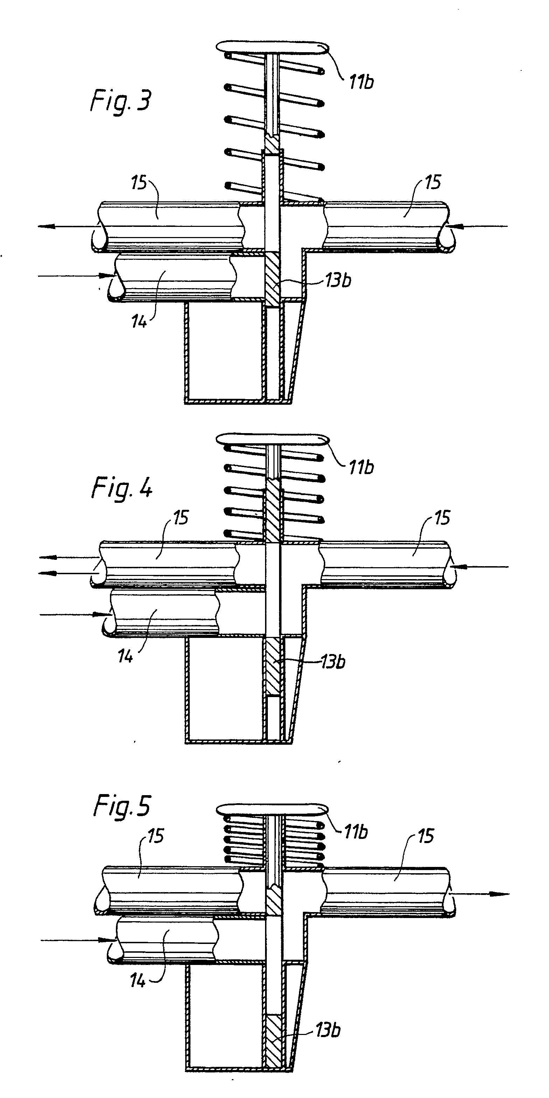

An alternative valve arrangement is schematically shown in fig 3-5, in which the regulating element is denoted by 1Ib and a sliding blocking element by 13b. In the same way as in fig 2, the mixture of anticoagulant and diluent can, by means of this valve, be fed from conduit 14 into conduit 15 to allow its mixing with the blood drawn up by the suction device. A further advantage of this valve arrangement is evident from fig 5, namely, the connection to the cardiotomy reservoir 3 can be completely blocked off whilst aforementioned mixture is fed out through conduit 15 towards the suction tip 12. This setting is selected when it is desired to flush the suction site (for example an operation wound) with the mixture.

In fig 1 conduit 14 leads into tube 14′, which is in communication with storage vessel 2, and conduit 15 leads into tube 15′ which is connected to the cardiotomy reservoir 3. The drawing up of the blood and said mixture is attained by means of the cardiotomy reservoir 3 being connected to a vacuum source (not shown) via a connection nipple 16. The complete mixture is fed in its entirity from the cardiotomy reservoir to the recirculation circuit 17. This is achieved by means of two valves 18 and 19 and a coupling 20, which are so arranged that the entire recirculation circuit may be disconnected from the rest of the system should it need to be exchanged. A further two valves are denoted by 21 and 22. These are used when the interruption of the blood treatment, i.e. recirculation, is desired and hence diversion of the treated blood from the intermediate storage vessel 7 to the final storage vessel 8. This occurs when the measuring device 9 registers an appropriate haematocrit value and/or Hb value.

By haematocrit is meant the quantity of red blood corpuscles (erythroucytes) expressed as a percentage volume of the total blood volume. Normal Hct is circa 45%. This can be compared with the definition of the Hb value, which indicates the concentration of haemaglobin, that is, the oxygene-carrying blood components, in the total blood volume expressed in g/l. A normal Hb value is circa 140-160 g/l.

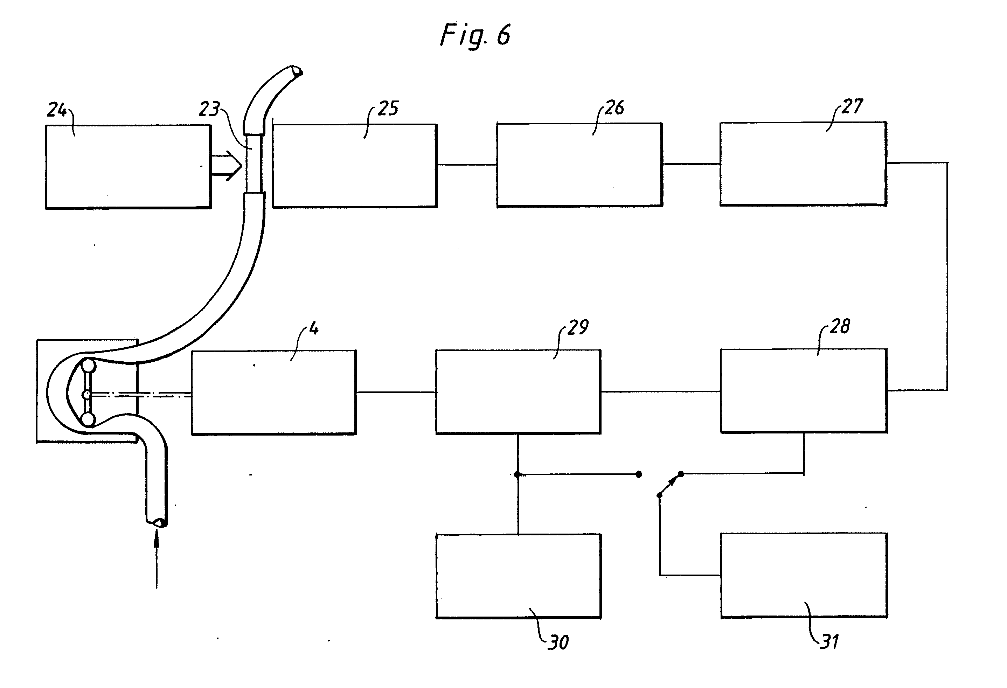

Fig 6 shows a block diagram of a preferred embodiment of a haematocrit measuring device or Hb measuring device according to the invention. Its principal of operation is based on measuring how much light is absorbed in a cuvette 23, which forms a part of the device 9 shown in fig 1. A gallium-aluminum-arsenic-diode, which emits infrared light with a maximum intensity of approximately 880 nm, is used as a suitable sender 24. Infrared light is chosen since the measuring in this range is less dependent on the oxygene content of the blood. So that background radiation does not unduly influence the measuring, the sender-diode's light is pulsated at a frequency of circa 33 kHz. Though this frequency is not critical, it should lie well above 50-100 Hz, a frequency range common to many interference sources. Since electro-acoustic phenomena can also create disturbance, a sender frequency above the normal audible range is selected. A receiver comprising a current amplifier is denoted by 25. The received signal is then transmitted to a bandpass filter 26, which is set to the same 33 kHz. It is this combination of sender and filter which reduces the system's susceptibility to interference. The absorption in a sample occurs as an exponential function. Thus, a logarithmic amplifier 27 is employed to obtain adequate signal sizes and a suitable measuring range. The signal from the logarithmic amplifier 27 is then connected to another amplifier 28 with offset control. The control is used to adjust the rest level of the amplifier's output. The output signal is compared in a comparator 29 with a reference value which can be adjusted as desired. When the signal from the amplifier 28 exceeds the reference value, the comparator 29 switches and sends a signal to the pump 4 (fig 1) so that it stops. At the same time, valve 21 is closed and valve 22 opened so that the treated blood can be transferred from the intermediate storage vessel 7 to the final storage vessel 8. As a reference value, a predetermined value from the patient's own blood sample can be used. Thus, before the treatment commences, a blood sample may be taken from the patient, placed in the cuvette 25 and its haematocrit value measured. Thereafter a reference value generator 30 is set proportionally to the measured value. The measured value and the reference value can be checked with the aid of a digital display 31.

TEST OF THE HAEMATOCRIT MEASURING DEVICE 9

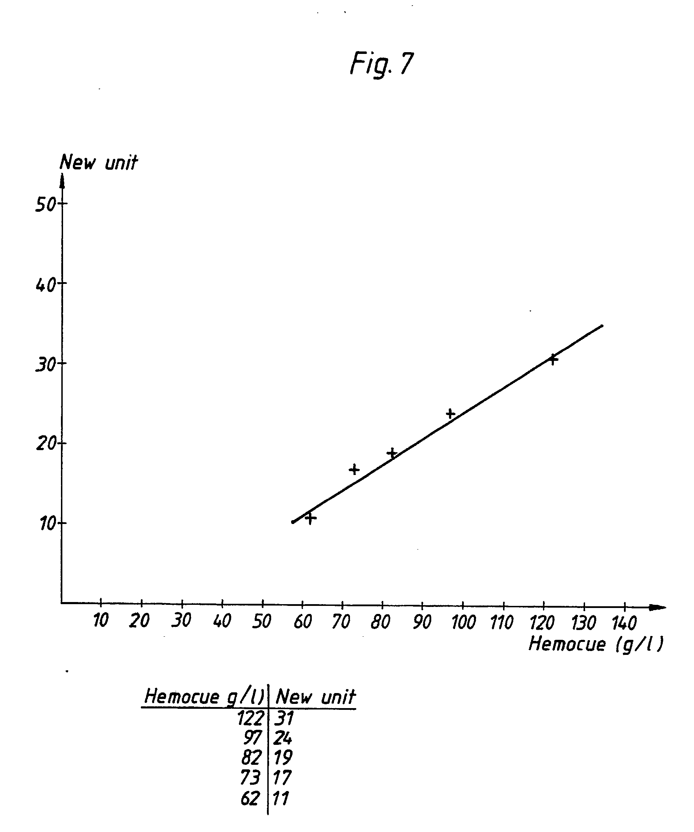

With the help of the system according to fig 6, the five values given in the table of fig 7 were measured, and compared with five measured values which came from the same sample and which were determined by means of an existing, appropriate Hb measurer HEMOCUE. The values measured using the system of the present invention can be said to be stated in a new unit. However, as can be seen from the diagram, a linear relationship exists with a normally adopted value of Hb, i.e. g/l. Thus, the measured values can simply be converted to the normally adopted unit g/l. Naturally, this can be carried out within the measuring device so that this shows directly the converted value. Alternatively, the conversion can be to the unit normally adopted for haematocrit, i.e. percent.

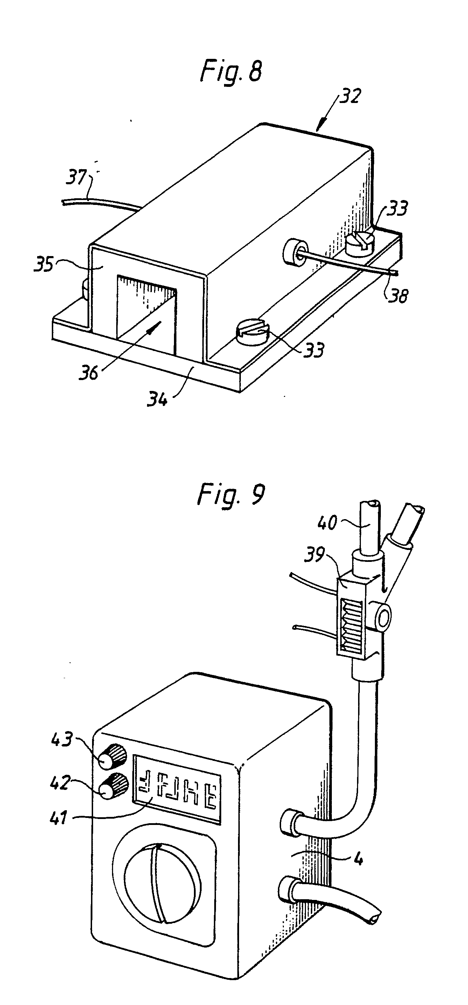

Accordingly, in fig 8 there is depicted a simple sketch of the prototype device used to establish the measured values according to fig 7. In this respect reference numeral 32 denotes a removable aluminum cover, which, with the help of screws 33, can be secured to a base plate 34. This base plate 34 together with a U-shaped beam 35 forms a passage 36 which is designed to receive the transparent plastic cuvette 23 shown in fig 6. The components 34 and 35 are manufactured from a suitable opaque plastic, for example black delrin. Finally, reference numerals 37 and 38 respectively denote connections to the gallium-aluminum-arsenic-diode and silicon detector of the measuring device.

Fig 9 shows how the haematocrit measuring device can be combined with a pump 4. The above-mentioned diode respectively detector can accordingly be built into a clamping arrangement 39, which tightly encloses the blood conduit 40. The pump casing is equipped with a display 41 to show the reference value respectively measured value. The reference value can be input by means of knob 42, whilst a second knob 43 is used to switch over the display from reference value to measured value and vice versa.

Naturally, the invention is not limited simply to the embodiments described above, but may be varied within the scope of the following claims. A suitable, alternative design of the suction device is shown by way of example in the concurrent Swedish patent application 89.02013-5.

Claims

1. An auto transfusion system for the collection, treatment and return of a patient's blood, comprising means for performing the respective steps, for example a suction device (1) for drawing up blood and, where present, any rinsing fluids from an operation site or similar, a source (2) of anticoagulant, a source (2) of diluent, a collection vessel (3) for the to-be-treated blood, a filter (5) for the removal of a filtrate comprising at least a quantity of the diluent, and a storage vessel (8) for finally treated blood, **characterized in** that the said sources consist of a common storage vessel (2) for a mixture of anticoagulant and diluent.

2. A system according to claim 1, **characterized in** that the said common storage vessel (2) is connected to the suction device (1) or to a point near the suction device on a conduit 15' leading therefrom.

3. A system according to claim 2, **characterized in** that the said connection is achieved via a manually operable valve (11,11a,11b), which is so dimensioned that in its open position it gives a maximum mixing ratio between blood and added fluid 1:1.

4. A system according to anyone of the preceding claims, **characterized by** a recirculation circuit (17) arranged between said collection vessel (3) and the storage vessel (8) for finally treated blood, said circuit (17) comprising said filter (5), a recirculation pump (4) and an intermediate storage vessel (7) for partially treated blood.

5. A system according to anyone of the preceding claims, **characterized by** a measuring device (9) for measuring the haematocrit- and/or Hb value of the treated blood.

6. A system according to claims 4 and 5, **characterized in** that the measuring device (9) is arranged in the recirculation circuit (17) preferably in connection with the recirculation pump (4).

7. A system according to claims 4 and 5, **characterized by** a valve (22) arranged between said intermediate storage vessel (7) and the storage vessel (8) for finally treated blood, said valve being opened manually or automatically when the measured blood value indicates that the treated blood is suitable for returning to the patient.

8. An auto transfusion system comprising a suction device (1) for drawing up blood and, where present, any rinsing fluids from an operation site or similar, a source (2) of anticoagulant, a source (2) of diluent, a collection vessel (3), a filter (5) for the removal of a filtrate comprising at least a quantity of the diluent, and a storage vessel (8) for finally treated blood, **characterized by** a measuring device (9) for measuring the haematocrit- and/or Hb value of treated blood.

9. A system according to claim 8 **characterized by** a recirculation circuit (17) arranged between said collection vessel (3) and the storage vessel (8) for finally treated blood, said circuit (17) comprising said filter (5), a recirculation pump (4) and an intermediate storage vessel (7) for partially treated blood.

10. A system according to claim 9 **characterized in** that the measuring device (9) is arranged in the recirculation circuit (17), preferably in connection with the recirculation pump (4).

11. A system according to claim 9 **characterized by** a valve (22) arranged between said intermediate storage vessel (7) and the storage vessel (8) for finally treated blood, said valve being opened manually or automatically when the measured blood value indicates that the treated blood is suitable for returning to the patient.

12. A system according to claim 8, **characterized in** that the said sources consist of a common storage vessel (2) for a mixture of anticoagulant and diluent.

13. A system according to claim 12, **char-**

**acterized in** that the said common storage vessel (2) is connected to the suction device (1) or to a point near the suction device on a conduit 15' leading therefrom.

14. A system according to claim 13, **characterized in** that the said connection is achieved by a manually operable valve (11,11a,11b), which is so dimensioned that in its open position it gives a maximum mixing ratio between blood and added fluid of 1:1.

15. A system according to either of claims 5 and 8, **characterized in** that said measuring device (9) is based on a measuring of infrared light passing through the blood.

16. A system according to claim 15, **characterized in** that said light is produced with the help of a sender (24), for example a diode, for pulsating light at a relatively high frequency, for example 33 kHz.

17. A system according to claim 16, **characterized in** that the light receiver (25), preferably via a current amplifier, is coupled to a bandpass filter (26), which is set to the same frequency as said sender.

18. A system according to claim 17, **characterized in** that said current amplifier is of a logarithmic type.

19. A system according to anyone of the preceding claims, **characterized by** means (29) for interrupting the blood's treatment when a suitable haematocrit- and/or Hb value has been reached.

20. A system according to claim 19, **characterized by** means (29) for comparing the measured haematocrit value and/or Hb value with a reference value, which is preferably based on a blood sample taken from the patient before treatment, and which is measured with the help of the same haematocrit- and/or Hb measuring device (9).

*Fig. 1*

*Fig. 2*

Fig. 3

15          15

11b

14          13b

Fig. 4

15          15

11b

14

13b

Fig. 5

15          15

11b

14

13b

Fig. 6

## Fig. 7

| Hemocue g/l | New unit |
|---|---|
| 122 | 31 |
| 97 | 24 |
| 82 | 19 |
| 73 | 17 |
| 62 | 11 |

## Fig. 8

## Fig. 9